# EUROPEAN PATENT APPLICATION

(11) **EP 4 834 229 A1**
(43) Date of publication of application: **04.11.2026**
(21) Application number: 25172925.7
(22) Date of filing: 28.04.2025
(51) Int. Cl.: A61B 5/349, A61B 5/352, A61B 5/363, A61B 5/00, G16H 50/20

(54) **SYSTEM AND METHOD FOR DETECTING A PHYSIOLOGICAL EVENT**

(71) Applicant: Koninklijke Philips N.V., 5656 AE Eindhoven (NL)
(72) Inventor: BONOMI, Alberto Giovanni, Eindhoven (NL)
(74) Representative: Philips Intellectual Property & Standards

(57) **Abstract**

A system and method for detecting a physiological event. One or more physiological signals for a subject and one or more configuration parameters for detection of the physiological event are obtained. An event detection model comprising a neural network is obtained and one or more modifications to operation of the event detection model are determined based on the one or more configuration parameters. The event detection model is used, applying the one or more modifications, to detect any instances of the physiological event based on the one or more physiological signals, and a user-perceptible output is provided by a feedback unit in response to detecting an instance of the physiological event.

## Description

### FIELD OF THE INVENTION

The invention relates to the field of detecting physiological events.

### BACKGROUND OF THE INVENTION

Hospital patients (e.g. patients in an intensive care unit) are continuously monitored in order to alert clinicians in case of serious adverse events, such as cardiac arrhythmias, which require prompt clinical intervention. Clinicians may be alerted to cardiac arrhythmias by the use of an arrhythmia detection system.

Arrhythmia detection systems typically process electrocardiography (ECG) signals to detect changes in rhythm. One issue with arrhythmia detection systems is the false alarm rate. False alarms may be a result of a poor signal-to-noise ratio. A high false alarm rate may lead to alarm fatigue among clinicians, resulting in slower responses from clinicians or even missed alarms. Many conventional arrhythmia detection systems allow a user to configure various parameters to achieve a desired alarming behavior which can impact the amount of false alarms.

In recent years, AI-based systems have been developed for ECG analysis and arrhythmia detection. These systems can provide better results than conventional arrhythmia detection systems; however, the internal processes of a machine-learning algorithm are difficult for a user to interpret. This makes it challenging for a user to configure the processing method of the machine-learning algorithm to achieve a desired output (such as a desired alarming behavior and thereby false alarm rate).

### SUMMARY OF THE INVENTION

The invention is defined by the claims.

According to examples in accordance with an aspect of the invention, there is provided a processing system for detecting a physiological event, the processing system being configured to: obtain one or more physiological signals for a subject; obtain a user input defining one or more configuration parameters for detection of the physiological event; obtain an event detection model trained to detect the physiological event based on the one or more physiological signals, wherein the event detection model comprises a neural network; determine, based on the one or more configuration parameters, one or more modifications relating to how the event detection model is used to detect the physiological event; process the one or more physiological signals, using the event detection model and applying the one or more modifications, to detect any instances of the physiological event; and in response to detecting an instance of the physiological event, control a feedback unit to provide a user-perceptible output indicating the detection of the instance of the physiological event.

In this way, the processing behavior of an event detection model is modifiable according to a desired user outcome, thus enabling a clinician to mitigate a risk of an incorrect output and to intervene to improve a likelihood of achieving an expected interpretation result. The one or more configuration parameters may indicate which information is to be used by the event detection model and/or how the information is to be used. Thus, the one or more modifications may modify the way in which the physiological signal(s) are processed by the event detection model and/or the way in which the output of the event detection model is used to detect instances of the physiological event.

In some examples, the physiological event may be an event relating to cardiac activity; for instance, the physiological event may be an R-peak or an arrhythmia event.

The event detection model may, for example, comprise a convolutional neural network, a U-net neural network, a residual neural network, a recurrent neural network, or a combination thereof.

In some examples, the one or more physiological signals comprises a plurality of electrocardiography signals, each electrocardiography signal having been acquired by a respective electrocardiography channel.

In some examples, the physiological event is an adverse clinical event.

For instance, the adverse clinical event may be an arrhythmia event, a critical event of low oxygen saturation, or a respiration or blood-pressure-related critical event.

In some examples, the neural network comprises at least one dense, convolutional, recurrent or residual layer configured to process the one or more physiological signals to output a probability of the physiological event.

In some examples, the one or more physiological signals comprises a plurality of physiological signals; and the one or more configuration parameters comprises a parameter defining a subset of the plurality of physiological signals to be taken into account for detection of the physiological event.

In some examples, the one or more physiological signals comprises a plurality of physiological signals; and the one or more configuration parameters comprises a parameter defining an importance ranking for each of the plurality of physiological signals taken into account for detection of the physiological event.

In some examples, the one or more modifications comprise one or more selection mechanisms to be added to the neural network, wherein the one or more selection mechanisms are configured to control the processing of the plurality of physiological signals by the neural network.

In some examples, the one or more selection mechanisms comprise a gating mechanism.

In some examples, the one or more selection mechanisms comprise a weighting mechanism.

In some examples, the one or more selection mechanisms comprise an attention mechanism.

In some examples, the one or more configuration parameters comprises a parameter indicative of a target output characteristic.

The parameter indicative of a target output characteristic may be any parameter from which a target output characteristic (such as a sensitivity/false alarm rate) may be determined. In some examples, the parameter indicative of a target output characteristic may be a detection threshold relating to at least one of the one or more physiological signals. For instance, where the physiological event is an event relating to cardiac activity, the detection threshold may be a QRS detection threshold, a threshold on an asystole duration, a threshold on a ventricular tachycardia duration, or a threshold on a width of a QRS complex.

In some examples, the neural network is configured to determine a probability of the physiological event; the processing system is configured to determine a probability threshold based on the parameter indicative of a target output characteristic; and an instance of the physiological event is detected in response to: the probability of the physiological event being greater than a probability of the physiological event not occurring; and the probability of the physiological event being greater than the probability threshold.

In some examples, the processing system is configured to determine the probability threshold by: determining a target number of instances of the physiological event over a first period of time, wherein the target number of instances is determined based on the parameter indicative of a target output characteristic; and iteratively repeating the steps of: identifying a number of detected instances of the physiological event over the first period of time; determining a difference between the target number of instances and the number of detected instances; and in response to the difference exceeding a predetermined difference threshold, modifying the probability threshold based on the difference, until the difference between the target number of instances and the number of detected instances fails to exceed the predetermined difference threshold.

In some examples, the processing system is further configured, in response to one or more conditions being met, control the feedback unit to provide an alarm.

According to examples in accordance with another aspect of the invention, there is provided a computer-implemented method for detecting a physiological event, the computer-implemented method comprising: obtaining one or more physiological signals for a subject; obtaining a user input defining one or more configuration parameters for detection of the physiological event; obtaining an event detection model trained to detect the physiological event based on the one or more physiological signals, wherein the event detection model comprises a neural network; determining, based on the one or more configuration parameters, one or more modifications relating to how the event detection model is used to detect the physiological event; processing the one or more physiological signals, using the event detection model and applying the one or more modifications, to detect any instances of the physiological event; and in response to detecting an instance of the physiological event, controlling a feedback unit to provide a user-perceptible output indicating the detection of the instance of the physiological event.

There is also provided a computer program product comprising computer program code means which, when executed on a computing device having a processing system, cause the processing system to perform all of the steps of the method described above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a system for detecting a physiological event, according to an embodiment of the invention;
Fig. 2 illustrates examples of how a convolutional neural network having a U-net architecture may be modified to selectively process a plurality of physiological signals;
Fig. 3 illustrates an example of an iterative process for determining a probability threshold; and
Fig. 4 illustrates a computer-implemented method for detecting a physiological event, according to an embodiment of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system and method for detecting a physiological event. One or more physiological signals for a subject and one or more configuration parameters for detection of the physiological event are obtained. An event detection model comprising a neural network is obtained and one or more modifications to operation of the event detection model are determined based on the one or more configuration parameters. The event detection model is used, applying the one or more modifications, to detect any instances of the physiological event based on the one or more physiological signals, and a user-perceptible output is provided by a feedback unit in response to detecting an instance of the physiological event.

Illustrative embodiments may, for example, be employed in physiological monitoring systems, such as electrocardiography analysis systems and alarm systems for adverse clinical events.

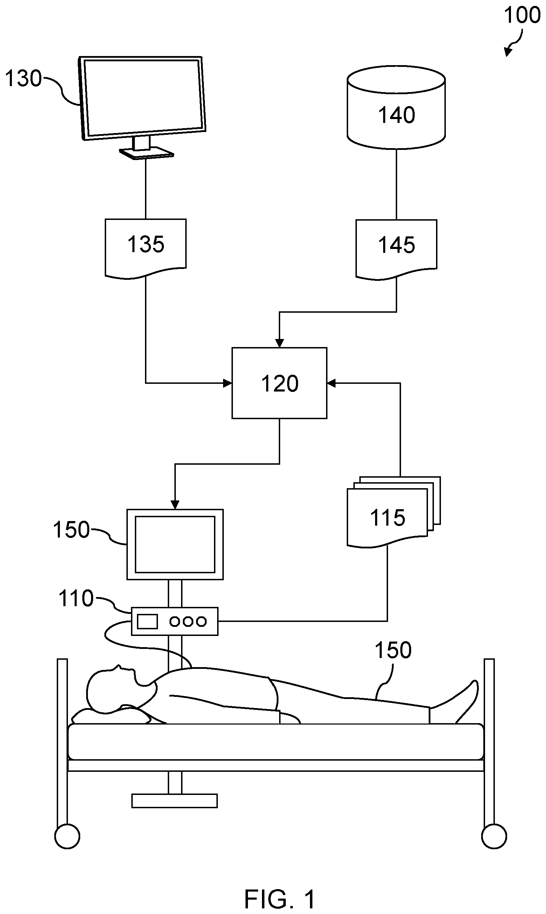

Fig. 1 illustrates a system 100 for detecting a physiological event, according to an embodiment of the invention. The system 100 comprises a sensor system 110, a processing system 120, a user interface 130, and a memory unit 140. The processing system 120 is, itself, an embodiment of the invention.

The physiological event to be detected by the system 100 may be any physiological event of clinical interest (including pathological, adverse and/or normal events). In some examples, the physiological event may be an event relating to cardiac activity; for instance, the physiological event may be an R-peak in the cardiac activity of a subject, or the physiological event may be an arrhythmia event, such as a ventricular tachycardia event, a ventricular fibrillation event or an asystole event. In other examples, the physiological event may relate to blood oxygen saturation; for instance, the physiological event may be a critical event of low blood oxygen saturation. In yet other examples, the physiological event may relate to blood pressure; for instance, the physiological event may be a blood-pressure-related critical event. In some examples, the detection of the physiological event may be used to determine whether a separate event has occurred, where the separate event is an adverse clinical event. For instance, the system 100 may be configured to detect R-peaks in order to detect an arrhythmia event.

The sensor system 110 is configured to acquire one or more physiological signals for a subject 150, and may comprise one or more sensors. In Fig. 1, the sensor system 110 is a bedside monitor; however, it is to be understood that the sensor system may be any type of sensor system capable of acquiring a one or more physiological signals that may be used to detect the physiological event. The skilled person will readily appreciate that the type(s) of sensors included in the sensor system will depend on the type of physiological event to be detected by the system. For instance, the sensor system may comprise one or more of: a plurality of electrocardiography electrodes, a photoplethysmography sensor and/or an arterial or cuff blood pressure sensor. In order to detect an event relating to cardiac activity, the sensor system may comprise a plurality of electrocardiography electrodes. In order to detect an event relating to blood oxygen saturation, the sensor system may comprise a photoplethysmography sensor. In order to detect an event relating to blood pressure, the sensor system may comprise a photoplethysmography sensor and/or an arterial or cuff blood pressure sensor, and a plurality of electrocardiography electrodes.

The processing system 120 is configured to obtain one or more physiological signals 115 for the subject 150 acquired by the sensor system 110. The one or more physiological signals may include one or more types of physiological signal (e.g. the one or more physiological signals may include a plurality of physiological signals of a same type and/or physiological signals of different types). The skilled person will readily appreciate that the type(s) of physiological signals will depend on the type(s) of sensors included in the sensor system and on the type of physiological event to be detected by the system. For instance, the one or more physiological signals may comprise any of: one or more electrocardiography signals, a photoplethysmography signal, an ambulatory blood pressure signal, and/or a respiration waveform. In order to detect an event relating to cardiac activity, the one or more physiological signals may comprise a plurality of electrocardiography signals, each electrocardiography signal having been acquired by a respective electrocardiography channel. In order to detect an event relating to blood oxygen saturation, the one or more physiological signals may comprise a photoplethysmography signal. In order to detect an event relating to blood pressure, the one or more physiological signals may comprise a photoplethysmography signal and/or an ambulatory blood pressure signal, and one or more electrocardiography signals.

In Fig. 1, the processing system 120 obtains the one or more physiological signals 115 directly from the sensor system 110; however, it is to be understood that the processing system may alternatively obtain the one or more physiological signals from another source storing the one or more physiological signals (e.g. the memory unit 140 or another memory unit).

The processing system 120 is further configured to obtain a user input 135 defining one or more configuration parameters for detection of the physiological event. The one or more configuration parameters may comprise any parameters defining how the one or more physiological signals are to be processed. For instance, the one or more configuration parameters may comprise one or more of: a parameter defining a subset of a plurality of physiological signals to be taken into account for detection of the physiological event (where the one or more physiological signals comprises a plurality of physiological signals); a parameter defining an importance ranking for each of a plurality of physiological signals taken into account for detection of the physiological event (where the one or more physiological signals comprises a plurality of physiological signals); and/or a parameter indicative of a target output characteristic (such as a target sensitivity).

For instance, where the physiological event is an event relating to cardiac activity, the one or more configuration parameters may comprise a parameter defining a subset of electrocardiography channels for which the electrocardiography signals should be taken into account for detection of the physiological event and/or a parameter defining an importance ranking for each electrocardiography channel (or each electrocardiography channel in the subset, where relevant). The one or more configuration parameters may additionally or alternatively comprise a parameter indicative of a target output characteristic, such a target sensitivity, a target false alarm rate, or a detection threshold, where the type of detection threshold depends on the type of physiological event to be detected. The detection threshold may be a threshold for detecting the physiological event in at least one of the one or more physiological signals by conventional means; an adjustment to the detection threshold would thus modify a sensitivity of detecting the event by conventional means. For instance, for detecting an R-peak, the detection threshold may be a QRS detection threshold, which defines a value above which a peak in an electrocardiography signal is to be detected as an R-peak. For detecting an asystole event, the detection threshold may be a threshold on asystole duration (i.e. the detection threshold is a duration beyond which an asystole event is to be detected). For detecting a ventricular tachycardia event, the detection threshold may be a threshold on ventricular tachycardia duration (i.e. the detection threshold is a duration beyond which a ventricular tachycardia event is to be detected). For detecting an abnormal ventricular beat, the detection threshold may be a threshold on a width of a QRS complex (i.e. such that an abnormal ventricular beat is to be detected if a QRS complex has a greater width than the detection threshold).

In Fig. 1, the user input 135 is acquired by the user interface 130. Any suitable user interface may be used to acquire the user input. In Fig. 1, the processing system 120 is configured to obtain the user input 135 directly from the user interface; alternatively, the processing system may obtain the user input from another source storing the user input (e.g. the memory unit 140).

The processing system 120 is further configured to obtain an event detection model 145. In Fig. 1, the event detection model is obtained from the memory unit 140. The event detection model is trained to detect the physiological event based on the one or more physiological signals and comprises a neural network. The neural network may have any suitable architecture, such as a convolutional neural network (CNN), a recurrent neural network (such as a Long Short-Term Memory, LSTM, network), a U-net, a residual neural network (RESNET) or a combination thereof.

Having obtained the one or more physiological signals 115 for the subject 150, the user input 135 and the event detection model 145, the processing system 120 is configured to determine, based on the one or more configuration parameters, one or more modifications relating to how the event detection model is used to detect the physiological event, and to process the one or more physiological signals, using the event detection model and applying the one or more modifications, to detect any instances of the physiological event. The one or more modifications may comprise any modification that would result in a desired change in the detection of the physiological event (as indicated by the one or more configuration parameters). For instance, the one or more modifications may comprise one or more modifications to the neural network and/or one or more modifications relating to how an output of the neural network is processed to detect any instances of the physiological event.

The one or more modifications determined based on the one or more configuration parameters depend on the type(s) of configuration parameters defined in the user input.

For instance, where the one or more configuration parameters comprises a parameter defining a subset of the plurality of physiological signals to be taken into account for detection of the physiological event and/or a parameter defining an importance ranking for each of the plurality of physiological signals taken into account for detection of the physiological event, the processing system 120 may be configured to determine one or more selection mechanisms to be added to the neural network. The application of the one or more modifications when using the event detection model may thus comprise adding the one or more selection mechanisms to the event detection model before processing the one or more physiological signals using the event detection model. The one or more selection mechanisms are configured to control the processing of the plurality of physiological signals by the neural network (e.g. to direct the processing of the plurality of physiological signals to a subset defined a configuration parameter or to physiological signals having a higher importance ranking).

Any suitable selection mechanism(s) may be used to control the processing of the plurality of physiological signals by the neural network. In some examples, the one or more selection mechanisms may comprise a gating mechanism configured to restrict the processing of one or more physiological signals. For instance, where the one or more configuration parameters comprises a parameter defining a subset of the plurality of physiological signals to be taken into account for detection of the physiological event, a gating mechanism may be used to restrict processing of any physiological signal excluded from the subset. Where the one or more configuration parameters comprises a parameter defining an importance ranking for each of the plurality of physiological signals taken into account for detection of the physiological event, a gating mechanism may be used to restrict processing of lower-ranked physiological signals (e.g. to restrict processing of the bottom half of the plurality of physiological signals according to importance ranking).

In some examples, the one or more selection mechanisms may comprise a weighting mechanism configured to weight how each physiological signal is processed. For instance, where the one or more configuration parameters comprises a parameter defining a subset of the plurality of physiological signals to be taken into account for detection of the physiological event, a weighting mechanism may be used to assign higher weights to the physiological signal(s) included in the subset. Where the one or more configuration parameters comprises a parameter defining an importance ranking for each of the plurality of physiological signals taken into account for detection of the physiological event, a weighting mechanism may be used to weight each physiological signal according to the importance ranking for the physiological signal.

In some examples, the one or more selection mechanisms may comprise an attention mechanism configured to highlight one or more physiological signals (i.e. to focus the processing of the neural network on the highlighted one or more physiological signals). For instance, where the one or more configuration parameters comprises a parameter defining a subset of the plurality of physiological signals to be taken into account for detection of the physiological event, an attention mechanism may be used to highlight the subset of physiological signals. Where the one or more configuration parameters comprises a parameter defining an importance ranking for each of the plurality of physiological signals taken into account for detection of the physiological event, an attention mechanism may be used to highlight higher-ranked physiological signals (e.g. to highlight the top half of the plurality of physiological signals according to importance ranking).

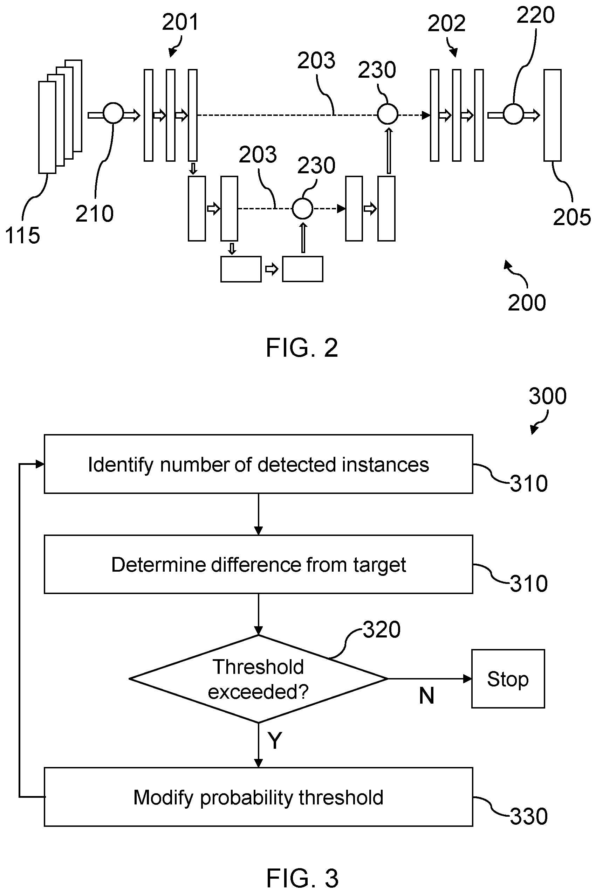

Fig. 2 illustrates examples of how a convolutional neural network having a U-net architecture 200 may be modified to selectively process the one or more physiological signals 115 (where the one or more physiological signals comprises a plurality of physiological signals) according to a parameter defining a subset of the plurality of physiological signals to be taken into account for detection of the physiological event and/or a parameter defining an importance ranking for each of the plurality of physiological signals taken into account for detection of the physiological event.

For instance, a gating or weighting mechanism 210 may be provided before a first group of convolutional layers 201. In this way, the convolutional neural network may process information solely carried by physiological signals that are not restricted by a gating mechanism or may process information primarily carried by physiological signals having a higher weight.

Additionally or alternatively, a gating or weighting mechanism 220 may be provided after a last group of convolutional layers 202. In this way, an output probability 205 of the convolutional neural network may be influenced solely by physiological signals that are not restricted by a gating mechanism or influenced primarily by physiological signals having a higher weight.

Additionally or alternatively, an attention mechanism 230 may be provided at each skip connection 203 of the convolutional neural network.

Returning to Fig. 1, where the one or more configuration parameters comprises a parameter indicative of a target output characteristic, the one or more modifications may comprise a modification to one or more probability rules used to detect the physiological event. For example, the processing system 120 may be configured to determine a probability threshold based on the parameter indicative of a target output characteristic. An instance of the physiological event may then be detected in response to a probability of the physiological event (determined by the neural network) being greater than a probability of the physiological event not occurring and greater than the probability threshold.

How the probability of the physiological event not occurring is determined may depend on the type of physiological event. In some examples, the probability of the physiological event not occurring may be complementary to the probability of the physiological event (i.e. the probability of the physiological event not occurring may be 1 - the probability of the physiological event). In other examples, a probability of other events that may have occurred instead of the physiological event may be determined for each other event, and the probability of the physiological event not occurring may be a total of the probabilities of each other event. For instance, where the physiological event is an R-peak, the neural network may determine an R-peak probability (i.e. the probability of the physiological event), a V-peak probability (a probability of a premature ventricular beat) and a P-peak probability (a probability of a paced beat). The probability of the physiological event (R-peak) not occurring may then be the sum of the V-peak probability and the P-peak probability.

The processing system 120 may be configured to determine the probability threshold based on the parameter indicative of a target output characteristic using any suitable technique. In some examples, the probability threshold may be determined using an iterative process. For instance, a target number of instances of the physiological event over a first period of time may be determined based on the parameter indicative of a target output characteristic, and the probability threshold may be iteratively modified until the detection of instances of the physiological event corresponds to the target. In this way, a higher detection threshold will result in a higher probability threshold, and a lower detection threshold will result in a lower probability threshold.

How the target number of instances of the physiological event over the first period of time is determined based on the parameter indicative of a target output characteristic will depend on the type of parameter indicative of a target output characteristic. For instance, where the parameter indicative of a target output characteristic is a detection threshold, the target number of instances of the physiological event may be determined by applying the detection threshold to at least one or more of the physiological signals acquired over the first period of time (e.g. to each physiological signal or a selected subset of physiological signals). For instance, where the physiological event is an R-peak and the parameter indicative of a target output characteristic is a QRS threshold, the target number of instances may be determined by applying the QRS threshold to at least one electrocardiography signal. In other words, the target number of instances is equal to the number of peaks in the electrocardiography signal during the first period of time that exceed the QRS threshold.

Fig. 3 illustrates an example of an iterative process 300 for determining the probability threshold based on the target number of instances of the physiological event. The iterative process begins at step 310 at which a number of detected instances of the physiological event over the first period of time is identified. The number of detected instances of the physiological event is the number of detected instances using a current value for the probability threshold. During a first iteration of the iterative process 300, the initial value for the probability threshold may be a random or default value. For instance, the initial value for the probability threshold may be 0.5.

At step 320, a difference between the target number of instances and the number of detected instances is determined.

At step 330, the difference between the target number of instances and the number of detected instances is compared to a predetermined difference threshold. The predetermined difference threshold may, for example, be a difference of 5-20% between the target number of instances and the number of detected instances.

In response to the difference between the target number of instances and the number of detected instances exceeding the predetermined difference threshold, the process proceeds to step 340, at which the probability threshold is modified based on the difference between the target number of instances and the number of detected instances, and steps 310 to 330 are repeated. For instance, if the target number of instances is greater than the number of detected instances, the probability threshold may be reduced, while, if the target number of instances is lower than the number of detected instances, the probability threshold may be increased.

In response to the difference between the target number of instances and the number of detected instances exceeding the predetermined difference threshold, the iterative process is complete, and the current value for the probability threshold is set as the value for the probability threshold.

Returning to Fig. 1, in response to detecting an instance of the physiological event, the processing system 120 is configured to control a feedback unit 150 to provide a user-perceptible output indicating the detection of the instance of the physiological event. Any suitable user-perceptible output may be provided, depending on the type of feedback unit. In Fig. 1, the feedback unit is a display device of the bedside monitor 110; the user-perceptible output may therefore be an image or a textual display. In some examples, where the physiological event is an adverse clinical event, the user-perceptible output may be an alarm (e.g. a sound and/or a light).

In some examples, detection of the physiological event may be used to determine whether an adverse clinical event has occurred. Thus, the processing system 120 may be configured to control the feedback unit 150 to provide an alarm in response to one or more conditions being met, where the one or more conditions are one or conditions relating to the detection of the physiological event for detection of an adverse clinical event.

For instance, where the physiological event is an R-peak, the one or more conditions may define a number of R-peaks over a predetermined period of time, a location of R-peaks and/or a type of R-peak (e.g. normal, ventricular, paced, etc.). In response to the one or more conditions being met, the processing system 120 may be configured to provide an alarm to indicate detection of an arrhythmia event.

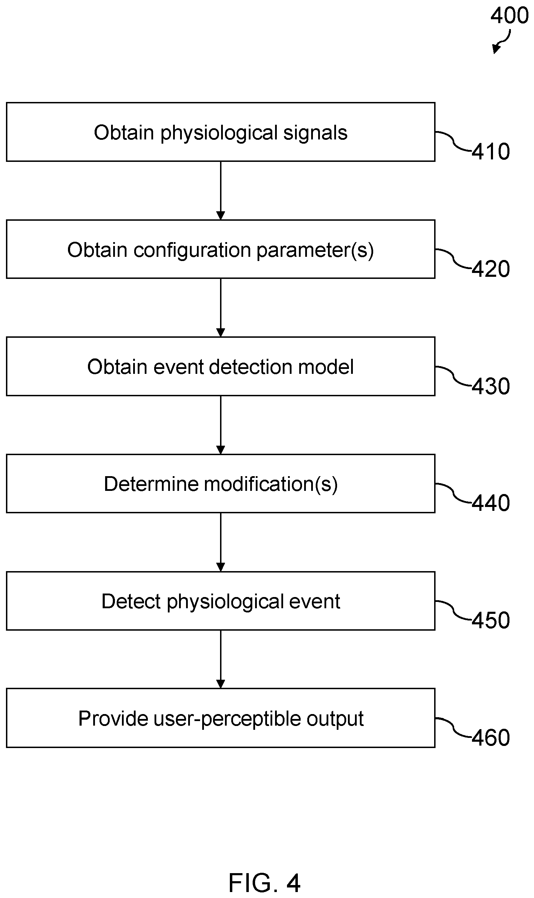

Fig. 4 illustrates a computer-implemented method 400 for detecting a physiological event.

The computer-implemented method 400 begins at step 410, at which one or more physiological signals for a subject are obtained.

At step 420, a user input defining one or more configuration parameters for detection of the physiological event are obtained.

At step 430, an event detection model is obtained. The event detection model is trained to detect the physiological event based on the one or more physiological signals, and comprises a convolutional neural network.

At step 440, one or more modifications relating to how the event detection model is used to detect the physiological event are determined based on the one or more configuration parameters.

At step 450, the one or more physiological signals are processed, using the event detection model and applying the one or more modifications, to detect any instances of the physiological event.

At step 460, in response to detecting an instance of the physiological event, a feedback unit is controlled to provide a user-perceptible output indicating the detection of the instance of the physiological event.

It will be understood that the disclosed methods are computer-implemented methods. As such, there is also proposed a concept of a computer program comprising code means for implementing any described method when said program is run on a processing system.

The skilled person would be readily capable of developing a processing system for carrying out any herein described method. Thus, each step of a flow chart may represent a different action performed by a processing system, and may be performed by a respective module of the processing system.

As discussed above, the system makes use of a processing system to perform the data processing. The processing system can be implemented in numerous ways, with software and/or hardware, to perform the various functions required. The processing system typically employs one or more microprocessors that may be programmed using software (e.g. microcode) to perform the required functions. The processing system may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processing system may be associated with one or more storage media such as volatile and non-volatile computer memory such as RAM, PROM, EPROM, and EEPROM. The storage media may be encoded with one or more programs that, when executed on one or more processing systems and/or controllers, perform the required functions. Various storage media may be fixed within a processing system or controller may be transportable, such that the one or more programs stored thereon can be loaded into a processing system.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processing system may be implemented by a single processing system or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Ordinal numbers (e.g. "first", "second" and so on) have been used purely to distinguish different elements from one another for the sake of clarity, and does not necessarily imply a specific order, importance, relationship, or presence of all numbered elements. Reference to a non-"first" (e.g. "second" or "third") element does not necessitate that a "first" element be present. The skilled person would be capable of relabeling any such elements as appropriate (e.g. relabeling a "second" element as a "first" element if only the second element is present).

Any reference signs in the claims should not be construed as limiting the scope.

## Claims

1. A processing system (120) for detecting a physiological event, the processing system being configured to:
obtain one or more physiological signals (115) for a subject (150);
obtain a user input (135) defining one or more configuration parameters for detection of the physiological event;
obtain an event detection model (145) trained to detect the physiological event based on the one or more physiological signals, wherein the event detection model comprises a neural network;
determine, based on the one or more configuration parameters, one or more modifications relating to how the event detection model is used to detect the physiological event;
process the one or more physiological signals, using the event detection model and applying the one or more modifications, to detect any instances of the physiological event; and
in response to detecting an instance of the physiological event, control a feedback unit (150) to provide a user-perceptible output indicating the detection of the instance of the physiological event.

2. The processing system (120) of claim 1, wherein the one or more physiological signals (115) comprises a plurality of electrocardiography signals, each electrocardiography signal having been acquired by a respective electrocardiography channel.

3. The processing system (120) of claim 1 or 2, wherein the physiological event is an adverse clinical event.

4. The processing system (120) of any of claims 1 to 3, wherein the neural network comprises at least one dense, convolutional, recurrent or residual layer configured to process the one or more physiological signals to output a probability of the physiological event.

5. The processing system (120) of any of claims 1 to 4, wherein:
the one or more physiological signals (115) comprises a plurality of physiological signals; and
the one or more configuration parameters comprises a parameter defining a subset of the plurality of physiological signals to be taken into account for detection of the physiological event.

6. The processing system (120) of any of claims 1 to 5, wherein:
the one or more physiological signals (115) comprises a plurality of physiological signals; and
the one or more configuration parameters comprises a parameter defining an importance ranking for each of the plurality of physiological signals taken into account for detection of the physiological event.

7. The processing system (120) of claim 5 or 6, wherein the one or more modifications comprise one or more selection mechanisms to be added to the neural network, wherein the one or more selection mechanisms are configured to control the processing of the plurality of physiological signals by the convolutional neural network.

8. The processing system (120) of claim 7, wherein the one or more selection mechanisms comprise a gating mechanism and/or a weighting mechanism (210, 220).

9. The processing system (120) of claim 7 or 8, wherein the one or more selection mechanisms comprise an attention mechanism (230).

10. The processing system (120) of any of claims 1 to 9, wherein the one or more configuration parameters comprises a parameter indicative of a target output characteristic.

11. The processing system (120) of claim 10, wherein:
the neural network is configured to determine a probability of the physiological event;
the processing system is configured to determine a probability threshold based on the parameter indicative of a target output characteristic; and
an instance of the physiological event is detected in response to:
the probability of the physiological event being greater than a probability of the physiological event not occurring; and
the probability of the physiological event being greater than the probability threshold.

12. The processing system (120) of claim 10 or 11, wherein the processing system is configured to determine the probability threshold by:
determining a target number of instances of the physiological event over a first period of time, wherein the target number of instances is determined based on the parameter indicative of a target output characteristic; and
iteratively repeating the steps of:
identifying a number of detected instances of the physiological event over the first period of time;
determining a difference between the target number of instances and the number of detected instances; and
in response to the difference exceeding a predetermined difference threshold, modifying the probability threshold based on the difference,
until the difference between the target number of instances and the number of detected instances fails to exceed the predetermined difference threshold.

13. The processing system (120) of any of claims 1 to 12, wherein the processing system is further configured, in response to one or more conditions being met, control the feedback unit (150) to provide an alarm.

14. A computer-implemented method (400) for detecting a physiological event, the computer-implemented method comprising:
obtaining one or more physiological signals (115) for a subject (150);
obtaining a user input (135) defining one or more configuration parameters for detection of the physiological event;
obtaining an event detection model (145) trained to detect the physiological event based on the one or more physiological signals, wherein the event detection model comprises a neural network;
determining, based on the one or more configuration parameters, one or more modifications relating to how the event detection model is used to detect the physiological event;
processing the one or more physiological signals, using the event detection model and applying the one or more modifications, to detect any instances of the physiological event; and
in response to detecting an instance of the physiological event, controlling a feedback unit (150) to provide a user-perceptible output indicating the detection of the instance of the physiological event.

15. A computer program product comprising computer program code means which, when executed on a computing device having a processing system, cause the processing system to perform all of the steps of the method (400) according to claim 14.
